# EUROPEAN PATENT APPLICATION

(11) **EP 4 852 008 A1**
(43) Date of publication of application: **09.12.2026**
(21) Application number: 26180822.4
(22) Date of filing: 25.05.2026
(51) Int. Cl.: G16H 20/30, G16H 40/63, G16H 50/30, G16H 50/70

(54) **METHOD AND SYSTEM FOR PERSONALIZED RECOMMENDATION OF CONTENT, SUCH AS FITNESS, MULTIMEDIA AND SIMILAR CONTENT**

(30) Priority: 03.06.2025 IT 202500012889
(71) Applicant: Technogym S.p.A., 47521 Cesena (FC) (IT)
(72) Inventor: TORTOLINI, Sofia, 47521 CESENA (FC) (IT); VALERIANI, Davide, 47521 CESENA (FC) (IT); DOVGANYUK, Rostyslav, 47521 CESENA (FC) (IT); MANCA, Giovanni, 47521 CESENA (FC) (IT)
(74) Representative: Tiburzi, Andrea

(57) **Abstract**

The present invention relates to a computer-implemented method (100) for processing and recommending personalized fitness content, wherein said method (100) is executed by a control unit (2), and wherein said method (100) comprises the steps of: acquiring (120) one or more combinations of content categories (51), wherein multimedia content (Cⱼ) is grouped into categories according to one or more combination criteria; acquiring a profiling (130) of said users (Uₖ) based on operational data into fitness profile groups (52), wherein said users (Uₖ) are grouped into said fitness profile groups (52) according to one or more grouping criteria; matching (140) each fitness profile group (52) with one or more combinations of content categories (51), so as to determine the preferences of each user (Uₖ); generating (150) a content recommendation (Cⱼ); and providing (160) via a communication device (311, 312,..., 31n) a recommendation of a multimedia content (Cⱼ) for each user (Uₖ); wherein said operational data (4) comprises training data relating to one or more users (Uₖ) and multimedia content usage data (Cⱼ) by said users (Uₖ); wherein said operational data (4) are acquired from one or more exercise devices (3; 31, 32,..., 3n), by means of which said users (Uₖ) performed gym training; and wherein each of said exercise devices (3; 31, 32,..., 3n) comprises said communication device (311, 312,..., 31n).

The present invention also relates to a system (1) for recommending personalized fitness content.

## Description

The present invention relates to a method and system for personalized recommendation of contents in the field of sports and fitness training.

### Field of invention

More specifically, the invention concerns a system and method of the mentioned type, studied and realized in particular to allow a *clustering* of users in various categories and allow for an optimal training experience, but which can be used for any circumstance in which it is necessary to automate recommendations and associations of content preferences, according to different needs or constraints.

In the following, the description will focus on applications in the field of sports training and fitness, but it is clear that it should not be considered limited to this specific use.

### Prior art

In the fitness and wellness field, technological evolution has led to significant changes in the way people train and track their progress.

The advent of wearable devices, smartphone apps, and connected gym equipment has created a digital ecosystem that generates a vast amount of data on users' physical activities.

This data comprises information such as heart rate, calories burned, workout duration, types of exercises performed, etc..

At the same time, there has been a proliferation of digital fitness content, such as workout videos, exercise programs, and articles on nutrition and healthy lifestyle.

This abundance of data and content offers unprecedented opportunities to personalize each user's fitness experience. However, simply having access to information does not automatically translate into an improved workout experience or better results for users. In fact, the overabundance of options can lead to confusion and indecision, making it difficult for users to choose the content best suited to their specific needs and goals.

Traditional recommendation systems used in other industries, such as e-commerce or video streaming, are not directly applicable to the fitness context. This is due to the complex and multidimensional nature of physical well-being, which involves factors such as current fitness, personal goals, training preferences, and physical limitations, to name a few.

A generic approach based only on past preferences or similarities with other users may not adequately capture an individual's specific needs at any given point in their fitness journey.

A technical challenge associated with recommendation systems in the fitness context is the difficulty of integrating and correctly interpreting data from different sources. Training information can come from wearable devices, *app* for smartphones, connected gym equipment, and manual user input. Each of these sources can use different data formats, have varying levels of accuracy, and provide partial information about a user's overall physical activity. Typically, a consistent and accurate user profile needs to be created that takes all these disparate data sources into account.

Another technical challenge concerns the dynamic nature of users' fitness needs. Unlike other domains where preferences tend to remain relatively stable over time, in fitness, users' goals and needs can change rapidly. For example, a user might shift from a focus on weight loss to a goal of gaining muscle mass, or might need to modify their workout regimen due to a temporary injury. Recommendation systems must be able to quickly adapt to these changes to provide relevant and useful suggestions.

Personalizing fitness content also raises safety and liability issues. Recommending a workout that is too intense or technically complex for a user's current fitness level could lead to injury. On the other hand, overly cautious or basic recommendations may not provide sufficient motivation for progress. Finding the right balance between challenge and safety is a significant challenge for fitness recommender systems.

In the context of gyms and fitness centers, a further complexity arises related to the need to balance personalized recommendations with efficient management of facility resources. An ideal recommendation system should not only suggest the best workout for the user, but also consider factors such as crowding in different areas of the gym, the availability of specific equipment, and even class or group scheduling.

The need to overcome one or more of these problems is felt in the sector.

### Purpose of the invention

In light of the above, it is therefore the purpose of the present invention to provide a personalized fitness content recommendation system capable of integrating data from different sources to create an accurate and complete user profile with multimedia sources.

Another purpose of the invention is to develop a method that dynamically adapts to the changing needs and goals of users over time.

It is also a purpose of the present invention to offer fitness content recommendations that effectively balance safety and effectiveness, considering the user's current fitness level.

Another aim of the invention is to optimize the use of available resources, both in terms of equipment and space in fitness facilities.

Another purpose of the invention is to provide multimedia educational and motivational content integrated into workout recommendations to enhance the overall user experience.

It is also the purpose of the present invention to develop a scalable system capable of handling large volumes of data and generating real-time recommendations for a large number of users.

### Object of the invention

It is, therefore, specific object of the present invention a computer-implemented method for processing and recommending personalized fitness content, wherein said method is executed by a control unit, and wherein said method comprises the steps of: acquiring one or more combinations of content categories, wherein multimedia content is grouped into categories according to one or more combination criteria; acquiring a profiling of said users based on operational data into fitness profile groups, wherein said users are grouped into said fitness profile groups according to one or more grouping criteria; matching each fitness profile group with one or more combinations of content categories, so as to determine the preferences of each user; generating a content recommendation; and providing via a communication device a recommendation of a multimedia content for each user; wherein said operational data comprises training data relating to one or more users and multimedia content usage data by said users; wherein said operational data are acquired from one or more exercise devices, by means of which said users performed gym training; and wherein each of said exercise devices comprises said communication device.

Always according to the invention, said profile groups may comprise at least one of: a group based on the type of training, such as cardio and the like; a group based on the training activity, such as running, rowing and the like; and a group based on the training objective, such as leg strengthening, weight loss and the like.

Still according to the invention, in said matching step the determination of the preferences of each user may take place by detecting occurrences between said one or more combinations of content categories and said fitness profile groups.

Preferably according to the invention, before said step of acquiring one or more combinations of content categories, it may comprise the step of acquiring said operational data, wherein said operational data may be acquired from said one or more exercise devices within a time interval.

Advantageously according to the invention, said combination criteria for determining said combinations of categories may be based on algorithms comprising algorithms for identifying recurring associations, such as Apriori, ECLAT and/or FP-Growth.

Still according to the invention, said category combinations may comprise one or more of the following categories: a goal category; a duration category; an experience level category; a target muscle category; and a workout type category.

Further according to the invention, said grouping criteria for determining said fitness profile groups may comprise distance-based clustering algorithms, such as K-means and qt-clustering.

Preferably according to the invention, said step of generating a multimedia content recommendation may comprise the sub-step of selecting a multimedia content based alternatively: on the probability weighted by said number of combinations; and/or on a random selection of a multimedia content.

Still according to the invention, said multimedia content may comprise at least one of: videos, such as musical exercise training videos; audio files; podcasts; videos with a single trainer instructing the workout; class videos, with participants; and audio or video description of the exercise.

Preferably according to the invention, said control unit may be a cloud server, remotely connected with said exercise devices, and said operational data may be stored in a database or storage unit, connected to said control unit, in which said fitness profile groups and said plurality of category combinations are stored, wherein said database or storage unit may be located on a remote server.

Advantageously according to the invention, said step of generating a recommendation may comprise the transmission of said recommendation through a data exchange interface of said control unit, wherein said data exchange interface may be connected to said database or storage unit.

Still according to the invention, said communication device may comprise a display.

Further according to the invention, one or more exercise devices may comprise: gym machines, such as stationary bikes, treadmills, rowing machines and the like; and/or wearable devices, such as smartwatches, heart rate straps, fitness trackers and the like.

It is also object of the present invention a system for recommending personalized fitness content, comprising: one or more exercise devices for performing exercises; a control unit, comprising a processing module, a data exchange interface, for allowing data input, connected to said processing module and for transmitting said data, and a memory containing instructions that, when executed by said processing module, cause said control unit to execute the steps of the method according to any one of the preceding claims; a database or storage unit, operatively connected to said control unit, in which said fitness profile groups and said plurality of category combinations are stored; wherein said control unit is connected to said exercise devices, for acquiring said operational data acquired within a time interval from one or more users.

Preferably according to the invention, said recommended content may be displayed via the communication device of the respective exercise device.

Still according to the invention, said data exchange interface may be an Application Programming Interface module.

Further according to the invention, said operational data may comprise: training data relating to one or more users and multimedia content usage data by said users; and/or information about the health of the users; and/or preferences and training objectives of each of said users.

Still according to the invention, said processing module may execute said method by means of algorithms, wherein said algorithms may comprise at least one artificial intelligence algorithm.

It is further object of the present invention a computer program comprising instructions that, when the program is executed by a computer, cause the computer to perform the steps of the method as described above.

It is further object of the present invention a computer-readable storage medium comprising instructions that, when executed by a computer, cause the computer to perform the steps of the method as described above.

### Brief description of the figures

The present invention will be now described, for illustrative but not limitative purposes, according to its preferred embodiments, with particular reference to the figures of the enclosed drawings, wherein:
figure 1 shows a block diagram of a system for recommending personalized fitness content, according to aspects of the present invention;
figure 2 shows a diagram showing the different user profile groups;
figure 3 is a diagram showing the combination of media content categories;
figure 4 shows a content recommendation or occurrence table showing the relationship between fitness profile groups and media content category combinations, according to one aspect of the present invention; and
figure 5 shows a flowchart of a method for providing personalized fitness content recommendations, in accordance with aspects of the present invention,

### Detailed description

In the various figures, similar parts will be indicated with the same numerical references.

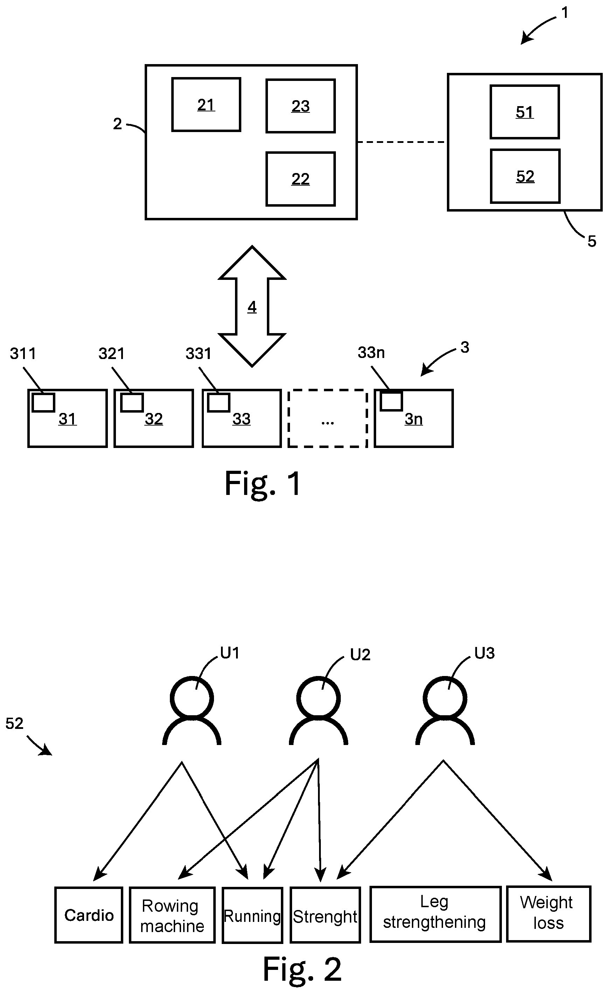

With reference to figure 1, a system for recommending personalized fitness content is illustrated, indicated overall with the numerical reference 1.

The system 1 comprises a control unit 2, connected to a set of exercise equipment 3 via a data exchange interface 22. The system 1 also comprises a database or storage unit 5 connected to (or included in) the control unit 2 for exchanging and saving data from the set of exercise equipment 3 via the data exchange interface 22.

The control unit 2 can be in the cloud and therefore remote from said exercise equipment 3.

In some embodiments, the data exchange interface 22 is an application programming interface (API) module for communicating (both receiving and transmitting) with each piece of equipment in the exercise equipment set 3. As seen in figure 1, the operational data 4 stream from said exercise equipment 3 is acquired by said data exchange interface 22.

The exercise equipment 3 in some embodiments may be exercise machines 31, 32,...,3n, such as a stationary bike, treadmill, rowing machine and the like, mobile devices associated with each user, such as a smartphone or a smartwatch, wearable devices, such as heart rate monitors, fitness trackers and the like, which, as is known, are equipped with screens and interfaces in general. The aforementioned exercise equipment 3 may be appropriately programmed with dedicate app.

Operational data stream 4 comprises both training data of each user Uₖ, and the multimedia contents enjoyed by each user Uₖ while doing the exercises.

As mentioned, for content Cⱼ it is referred to digital fitness content, such as workout videos, exercise programs, etc.. Specifically, and by way of example, such digital content may include videos showing a trainer providing workout instructions, videos showing a class of users performing a workout, or sequential exercise programs with related instructions (e.g., number of repetitions, number of sets, execution time, etc.). Such content may also include educational content relating to the benefits of physical exercise, or even nutritional advice.

Therefore, multimedia content may generally comprise videos, such as music workout videos,audio files, podcasts, videos with a single trainer who instructs on the workout,class video, with participants, audio or video description of the exercise.

Still referring to figure 1, the control unit 2 also comprises a processing module 21 and a related memory 23. The processing module 21 executes instructions stored in the memory module 23 to process the operating data 4 received through the data exchange interface 22.

Furthermore, always by means of the exercise equipment 3, the user can interact with the system 1, selecting or validating the chosen contents by means of communication devices 311, 312,..., 31n, with which each exercise equipment 31, 32,..., 3n is equipped, such as a touch on screen display and the like.

The exercise equipment set 3 may comprise, as mentioned, exercise machines 31, 32, 33,..., 3n. Each exercise machine 3 is configured to collect training data from users Uₖ during the training sessions and transmit them to the control unit 2 via the stream of operational data 4.

In some embodiments, the stream of the operating data 4 can be realized by means of a transmission module integrated in the exercise equipment 3, or an external module connected to the equipment 3 itself, configured to communicate via the data exchange interface 22.

The processing module 21 is configured to process the received training data according to the instructions stored in the memory 23, to generate personalized fitness content recommendations. These recommendations can be displayed to users via the exercise equipment 3ᵢ, as mentioned above by means of communication devices 311, 312,..., 31n.

The storage unit 5 of the system 1 stores and manages the information of each of the said users Uₖ, the contents of combinations of content categories 51 and fitness profile groups 52, which are processed by said control unit 2, in particular by said processing module 21.

In some embodiments the control unit 2 is enabled both to acquire the operational training data 4 via the data exchange interface 22 of one or more users Uₖ from exercise equipment 3, such as gym machines 3ᵢ, both to the storage of the aforementioned data in the storage unit 5.

The combinations of content categories 51 and fitness profile groups 52 are processed by system 1 to generate recommendations for users Uₖ.

The profile groups 52 are groupings referring to users Uₖ according (in the embodiment described) types of training sessions previously performed, which track the users' habits and/or objectives to be achieved.

For example, the habits of users Uₖ may comprise previous training habits and specific preferences, such as running, cycling, muscle strength, etc., and can be characterized with parameters such as exercise duration, muscles to be involved, type of training, and the like. Instead, with regards to the objectives to be achieved (i.e. the *"needs"* or need), there can be mentioned, by way of example only, weight loss, increased muscle strength, increased aerobic capacity, etc.

Referring to figure 2, a system diagram is illustrated, which schematically shows the user Uₖ profiling based on fitness activities in fitness profile groups 52. The diagram represents three users U1, U2, and U3 and their relationships with various fitness profiles, such as those already mentioned above. For example, user U2 belongs to the profile group that includes the profiles "rowing", "running" and "strength".

In general "fitness activity" refers to a set of information that a user Uₖ performs, the type of exercise machines 3 he uses, the objective he wants to pursue.

The fitness profile groups 52 comprise, as mentioned above, different types of fitness activities such as cardio, rowing, running, strength, leg strengthening, and weight loss. These categories represent groupings based on the type of workout, training preferences (e.g., workout duration, number of training sessions per week), and/or training goal.

The diagram in figure 2 shows how users U1, U2, and U3 are connected or connectable to multiple fitness profile groups 52 through directional arrows, which thus compose the fitness profile groups 52, indicating their participation or association with different types of activities. The connections between users U1, U2, U3 and the fitness profile groups 52 are represented by lines branching out from each user Uₖ towards their respective profiles.

Still referring to figure 2, a user Uₖ can be profiled both in the group based on the type of training "cardio" and in the group of fitness profiles 52 based on the training goal "weight loss". This multiple profiling allows the system 1 to create a heterogeneous profile of each user Uₖ more comprehensive and detailed, which can be used to provide more personalized and relevant fitness content recommendations.

User Uₖ profiling in fitness profile groups 52 is a dynamic process, which can be updated according to a pre-set period of time *ΔT,* through the processing module 21, based on the most recent training activities performed by users Uₖ, ensuring that content recommendations remain relevant and tailored to the same users' Uₖ changing preferences and fitness goals.

The combinations of categories 51 refer to media content preferences Cⱼ of each user Uₖ, determined based on averages and frequencies of use of the same multimedia contents and similar metrics, as better described below.

In one embodiment, the user Uₖ profiling can be achieved through statistical techniques such as "clustering", where the elements are grouped based on their mutual distance and therefore whether or not they belong to a set depends on how much the element (i.e., the multimedia content Cⱼ) under consideration is distant from the set itself. Examples of well-known distance-based clustering algorithms are K-means and qt-clustering.

According to aspects of the invention, the system 1 provides the possibility of modifying the assignment/membership of a user Uₖ to one or more fitness profile groups 52, based on changes that may occur over time, also considering that the activities carried out by each user may change over time Uₖ and the exercise equipment 3 used.

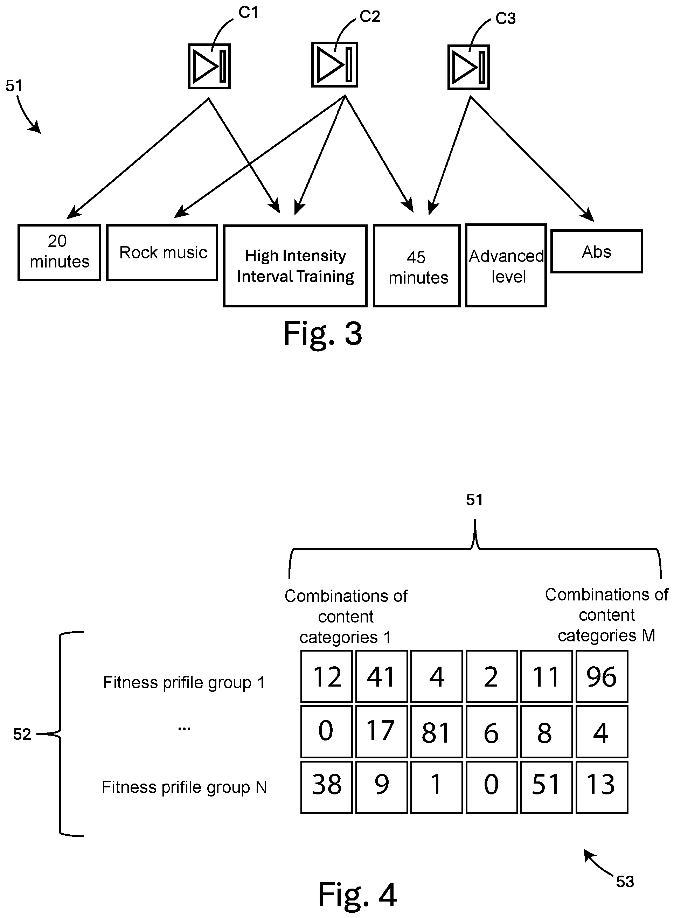

Referring to figure 3, a system diagram showing the combinations of content categories 51 is illustrated. The diagram represents three contents C₁, C₂, and C₃ and their relationships with various combinations of content categories 51, such as those already mentioned above.

The content category combinations 51 are processed by the processing module 21, in order to group the categories Cⱼ, in groups comprising statistical combinations of the above category combinations 51.

In one embodiment, algorithms for identifying recurring associations, such as Apriori, ECLAT, or FP-Growth, are used to create combinations 51 of the categories.

Creating these combinations allows organizing the multimedia content Cⱼ into more specific and relevant groups.

For example, a possible combination could comprise content Cⱼ that belong to both the "abs" goal category and the "20-minute" duration category, thus creating a group of content suitable for users looking for quick workouts to strengthen their abdominal muscles.

The system 1 may also comprise a customization interface (not shown in the figure), which allows for personalized review or supervision of content to users, for example by a coach or personal trainer via a messaging service (not shown in the figure).

The operation of the personalized fitness content recommendation system 1 can be as follows.

The processing module 21 is programmed to manage the computational aspects and operate the system 1, while the data exchange interface 22 manages, as mentioned, the interactions with the set of exercise equipment 3 and the storage unit 5.

In some embodiments, the processing module 21 and the data exchange interface 22 may be arranged in a single processor/processor.

When a user Uₖ is connected to system 1 with an exercise device 3 (i.e., a gym machine or a worn smartphone), the latter provides input data to system 1 for processing and generating recommendations.

As mentioned, in addition to the exercise machines 3, the data to be processed may come from a plurality of sources, such as wearable devices, such as smartwatches or headphones (not shown in the figures), equipped with biometric sensors to acquire and track so-called training indoor and outdoor data.

The system 1 acquires, by means of said exercise equipment 3, data which also comprises information on the health of the users, as well as preferences and training goals of each user Uₖ.

The transfer of this data can take place via common wireless protocols such as Bluetooth^{®} and the like.

The acquired data is used by the processing module 21 to profile the users and process the combinations of content categories 51 and profile groups 52, stored in the storage unit 5.

As mentioned, the storage unit 5 of the system 1 stores and manages the information of each of the said users Uₖ, the contents of said combinations of content categories 51 and profile groups 52.

The content category combinations 51 made on the contents Cⱼ may include, by way of example:
- a goal category;
- a duration category;
- an experience level category;
- a category of target muscles; and
- a training type category.

In other embodiments, additional and different categories/parameters may be used.

After a definable data collection period, for example, initially six months, followed by shorter update periods, it is possible to determine usage statistics for category combinations 51 from different user profile groups 52. This information can be presented in a tabular form.

In particular, with reference to figure 4, a table of occurrences 53 is illustrated, in which the rows represent different fitness profile groups 52 and the columns represent the different combinations of content categories 51. In each cell of the table of occurrences 53 the number of times that users Uₖ of a specific fitness profile group 52 has consumed contents belonging to each content combination 51.

The number in each cell of the occurrence table 53 is calculated by adding together the views of each piece of content from that combination of content categories 51 for each user in a profile group 52.

Therefore, always referring to figure 4, for each pair of fitness profile groups 52 and combinations of content categories 51, the processing module 21 calculates the number of views of each content Cⱼ belonging to that combination/cell of content categories 51 watched/played/listened to by all users Uₖ of that group of fitness profiles 52 in a predefined period of time.

The occurrence table 53 is updated weekly (or in a different time interval), through a scheduled processing.

The system also uses explicit and implicit feedback from users to refine content Cⱼ recommendations. Explicit feedback may include direct ratings provided by users Uₖ, while implicit feedback can be inferred from user viewing behavior of the users Uₖ and interactions with contents Cⱼ.

The occurrence table 53 in figure 4 is updated, as mentioned, at regular intervals *ΔT,* e.g. weekly (or different time interval), through a scheduled processing. This regular update ensures that the resulting recommendations remain relevant and reflect the most recent preferences of Uₖ users and content consumption trends Cⱼ.

The numerical values, appropriately added as shown in the figure, allow system 1 to evaluate and identify the most appropriate combinations of content categories 51 to recommend to users Uₖ belonging to certain fitness profile groups 52.

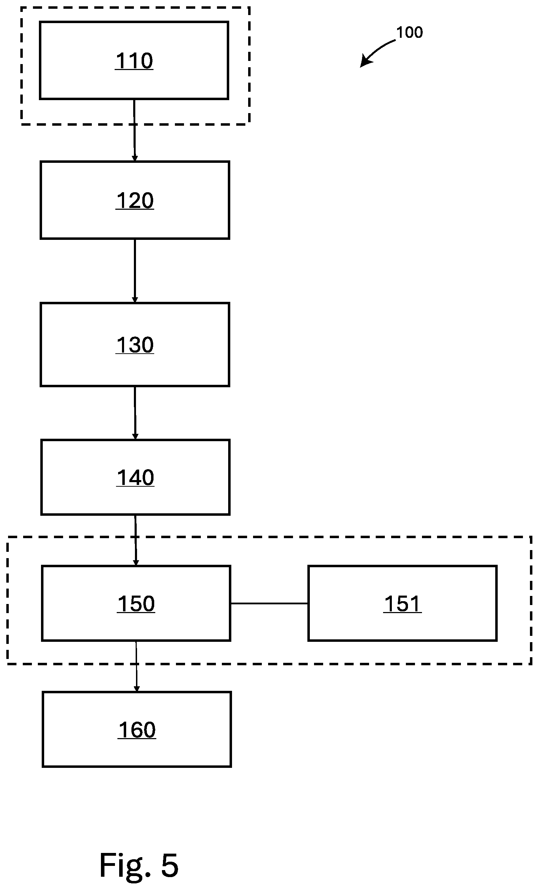

The memory 23 of the control unit 2 contains instructions that, when executed by processing module 21, cause control unit 2 to execute the steps of a method 100 for recommending personalized fitness content (of which flowchart is shown in figure 5).

The 100 method of profiling the user Uₖ is based on the training activities performed to assign users to the appropriate fitness profile groups 52.

This process allows for flexible profiling of user preferences and activities, allowing each user Uₖ to be associated with multiple fitness profiles simultaneously.

Referring now to figure 5, the flowchart of the method 100 for generating personalized fitness content recommendations is illustrated. The method 100 comprises a sequence of steps, which are further described below.

The method 100 initially provides for an acquisition step 110, in which the operational data 4 are acquired from one or more exercise devices 31, 32,...,3n regarding the training activities of the users Uₖ.

The operational data 4 is acquired and stored in the storage unit 5, which can be solid state or cloud based, to create user profiles Uₖ, based on their training patterns and preferences.

Data is acquired over a period of time *ΔT,* which in one embodiment is 28 days, although other acquisition intervals may be possible.

The method 100 then provides for a step 120 in which one or more combinations of content categories 51 are acquired or detected or determined, based on the operational data.

Subsequently, there is a step 130, in which certain groups of fitness profiles 52 are acquired or detected. In this phase, the users Uₖ are grouped into so-called fitness profile groups 52 according to one or more grouping criteria. This step is performed, for example, by executing an association algorithm.

The method 100 subsequently provides a matching step 140, in which each group fitness profiles group 52 is associated with one or more combinations of content categories 51, based on the preferences of each user Uₖ acquired in said time interval *ΔT.* In this step, the processing module 21 selects a cell in the occurrence table 53.

Finally, the method 100 comprises a content Cⱼ recommendation generation step 150 available followed by the step of providing 160 via a communication device 311, 312,..., 31n of the respective exercise equipment 3 a recommendation of a multimedia content Cⱼ* for each user Uₖ.

The content recommendation step 150 may include a sub-step for the selection 151 of a specific content Cⱼ^{*} among those contents Cⱼ belonging to that combination of content categories (detected by step 120) for his fitness profile group (detected by step 130).

The display device may be, for example, a screen or the touch-on-screen display of gymnastic equipment 3.

In one embodiment, the available content Cⱼ recommendation generation step 150 comprises a customization sub-step 151, in which a particular content C j^{*} is selected among the contents Cⱼ available. At this step 151, customization filters can be applied to the contents Cⱼ selected. Personalization filters are configured to adapt the set of contents Cⱼ to the specific characteristics of each user Uₖ. This adaptation is based on physiological parameter thresholds, performance levels, stated objectives, usage history or behavioral groupings derived from operational data 4. The application of filters takes place locally on the user's Uₖ terminal device or remotely on dedicated servers, depending on the system architecture.

The identification process 151 is preferably optimized through the use of data mining techniques or machine learning algorithms, configured to recognize content Cⱼ consumption patterns.

The processing module 21 can be implemented in various ways, depending on the specific needs of the platform and the users:
- implementation on Digital Electronic Circuits: the module can be incorporated into dedicated hardware, such as microprocessors or FPGAs (Field-Programmable Gate Arrays), for fast and efficient data processing;
- software and firmware: the algorithm can 5be programmed as software running on computers or mobile devices;
- hybrid solutions: a combination of hardware and software, where the algorithm runs on a central server and the user interfaces are managed by local devices;
- Cloud computing: The algorithm can be implemented in a cloud computing environment, enabling large-scale data access and processing with high availability and scalability.

### Advantages

An advantage of the present invention is to provide a personalized fitness content recommendation system that can integrate data from various sources, including connected gym equipment, to create an accurate and comprehensive user profile.

Another advantage of the present invention is to develop a method that dynamically adapts to the changing needs and goals of users over time, taking into account changes in training preferences, fitness goals, and physical condition.

It is also an advantage of the present invention to offer fitness content recommendations that effectively balance safety and effectiveness, considering the user's current fitness level and suggesting appropriate workouts that stimulate progress without risk of injury.

It is also an advantage of the present invention to provide educational and motivational content integrated into the workout recommendations, improving users' understanding and engagement in their fitness journey.

Another advantage of the present invention is that it implements meaningful metrics to accurately measure the impact of the recommendations on users' progress toward their fitness goals, providing valuable feedback to both users and fitness facility managers.

While aspects of the invention have been described in terms of various specific embodiments, it is apparent to those skilled in the art that many modifications, changes, and omissions are possible without departing from the spirit and scope of the claims. Furthermore, unless otherwise specified herein, the order or sequence of any process or method steps may be varied or re-sequenced in accordance with alternative embodiments.

Reference has been made in detail to embodiments of the disclosure, one or more examples of which are illustrated in the drawings. Each example is provided for illustrative purposes of the disclosure and not as a limitation of the disclosure. Indeed, it is apparent to those skilled in the art that various modifications and variations may be made to the present disclosure without departing from the scope or spirit of the disclosure. Reference throughout the disclosure to "an embodiment" or "an embodiment" or "some embodiments" means that the particular feature, structure, or characteristic described in connection with an embodiment is included in at least one embodiment of the subject matter described. Therefore, the appearance of the phrase "in an embodiment" or "in one embodiment" or "in some embodiments" at various points in the disclosure does not necessarily refer to the same embodiment(s). Furthermore, the particular features, structures, or characteristics may be combined in any suitable manner in one or more embodiments.

When elements of various embodiments are introduced, the terms "a," "one," "the," and "said" are intended to indicate that one or more elements are present. The terms "comprising," "including," and "having" are intended to be inclusive and mean that there may be additional elements beyond those listed.

The subject-matter described herein may be implemented in digital electronic circuits, cloud computing, software, firmware, or hardware, including the structural means described in this specification and their structural equivalents, or in combinations thereof. The subject-matter described herein may be implemented as one or more computer program products, such as one or more computer programs tangibly embedded in an information medium (e.g., a machine-readable storage device), or embedded in a propagated signal, for execution by, or to control the operation of, data processing equipment (e.g., a programmable processor, a computer, or multiple computers). A computer program (also known as a program, software, software application, or code) may be written in any form of programming language, including compiled or interpreted languages, and may be distributed in any form, including as a stand-alone program tool or as a module, component, subroutine, or other unit suitable for use in a computing environment. A computer program does not necessarily correspond to a file. A program can be stored in a portion of a file containing other programs or data, in a single file dedicated to the program in question, or in multiple coordinated files (e.g., files storing one or more modules, subprograms, or portions of code). A computer program can be distributed to run on one computer or multiple computers at a site, or distributed across multiple sites and interconnected by a communications network.

The processes and logic flows described in this disclosure, including the method steps of the subject matter described herein, may be executed by one or more programmable processors that execute one or more computer programs to perform the functions of the subject matter described herein by operating on input data and generating output. The processes and logic flows may also be executed, and the apparatus of the subject matter described herein may be implemented, as special-purpose logic circuits, such as a field-programmable gate array (FPGA) or an application-specific integrated circuit (ASIC).

Processors suitable for executing a computer program include, but are not limited to, general-purpose and special-purpose microprocessors, as well as one or more processors of any type of digital computer. Typically, a processor will receive instructions and data from read-only memory, random-access memory, or both. The essential elements of a computer are a processor to execute instructions and one or more memory devices to store instructions and data. Typically, a computer will also include, or be operationally coupled to receive data from or transfer data to, one or more mass storage devices to store data, such as magnetic disks, magneto-optical disks, or optical disks. Suitable information media for storing computer program instructions and data include all forms of non-volatile memory, including but not limited to semiconductor memory devices (e.g., EPROM, EEPROM, and flash memory devices); magnetic disks (e.g., internal hard drives or removable disks); magneto-optical disks; and optical disks (e.g., CD and DVD discs). The processor and memory may be integrated or embedded in special-purpose logic circuits.

To enable interaction with a user, the subject matter described herein may be implemented on a computer equipped with a display device, such as a CRT (cathode ray tube) or LCD (liquid crystal display) monitor, to display information to the user, a keyboard, and a pointing device (e.g., a mouse or trackball) through which the user can provide input to the computer. Other types of devices may also be used to provide interaction with a user. For example, the feedback provided to the user may be any form of sensory feedback (e.g., visual feedback, auditory feedback, or tactile feedback), and input from the user may be received in any form, including acoustic, vocal, or tactile input.

The techniques described herein may be implemented using one or more modules. As used herein, the term "module" refers to processing software, firmware, hardware, and/or various combinations thereof. At a minimum, however, modules should not be construed as software not implemented in hardware, firmware, or recorded on a non-transitory processor-readable recordable storage medium (i.e., modules are not software per se). Indeed, the term "module" should be interpreted to always include at least one piece of physical, non-transitory hardware, such as a portion of a processor or computer. Two different modules may share the same physical hardware (for example, two different modules may use the same processor and network interface). The modules described herein may be combined, integrated, separated, and/or duplicated to support various applications. Additionally, a function described herein as being performed on a particular module may be performed on one or more other modules and/or by one or more other devices instead of or in addition to the function performed on the particular module. Additionally, modules can be deployed on multiple devices and/or other local or remote components. Furthermore, modules can be moved from one device and added to another, and/or included in both devices.

The subject-matter described herein may be implemented in a computer system that includes a back-end component (e.g., a data server), a middleware component (e.g., an application server), or a front-end component (e.g., a client computer with a graphical user interface or a web browser through which a user can interact with an implementation of the subject matter described herein), or any combination of such back-end, middleware, and front-end components. The system components may be interconnected by any form or means of digital data communication, such as a communications network. Examples of communications networks include a local area network ("LAN") and a wide area network ("WAN"), such as the Internet.

The present invention has been described for illustrative but not limitative purposes, according to its preferred embodiments, but it is to be understood that modifications and/or changes can be introduced by those skilled in the art without departing from the relevant scope as defined in the enclosed claims.

## Claims

1. Computer-implemented method (100) for processing and recommending personalized fitness content, wherein said method (100) is executed by a control unit (2), and wherein said method (100) comprises the steps of:
acquiring (120) one or more combinations of content categories (51), wherein multimedia content (Cⱼ) is grouped into categories according to one or more combination criteria;
acquiring a profiling (130) of said users (Uₖ) based on operational data into fitness profile groups (52), wherein said users (Uₖ) are grouped into said fitness profile groups (52) according to one or more grouping criteria;
matching (140) each fitness profile group (52) with one or more combinations of content categories (51), so as to determine the preferences of each user (Uₖ);
generating (150) a content recommendation (Cⱼ); and
providing (160) via a communication device (311, 312,..., 31n) a recommendation of a multimedia content (Cⱼ) for each user (Uₖ);
wherein said operational data (4) comprises training data relating to one or more users (Uₖ) and multimedia content usage data (Cⱼ) by said users (Uₖ);
wherein said operational data (4) are acquired from one or more exercise devices (3; 31, 32,..., 3n), by means of which said users (Uₖ) performed gym training; and
wherein each of said exercise devices (3; 31, 32,..., 3n) comprises said communication device (311, 312,..., 31n).

2. Method (100) according to claim 1, **characterized in that** said profile groups (52) comprise at least one of:
a group based on the type of training, such as cardio and the like;
a group based on the training activity, such as running, rowing and the like; and
a group based on the training objective, such as leg strengthening, weight loss and the like.

3. Method (100) according to any one of the preceding claims, **characterized in that** in said matching step (140) the determination of the preferences of each user (Uₖ) takes place by detecting occurrences between said one or more combinations of content categories (51) and said fitness profile groups (52).

4. Method (100) according to any one of the preceding claims, **characterized in that** before said step of acquiring (120) one or more combinations of content categories (51), it comprises the step of acquiring (110) said operational data (4),
wherein said operational data (4) is acquired from said one or more exercise devices (3; 31, 32,..., 3n) within a time interval (*ΔT*).

5. Method (100) according to any one of the preceding claims, **characterized in that** said combination criteria for determining said combinations of categories (51) are based on algorithms comprising algorithms for identifying recurring associations, such as Apriori, ECLAT and/or FP-Growth.

6. Method (100) according to any one of the preceding claims, **characterized in that** said category combinations (51) comprise one or more of the following categories:
- a goal category;
- a duration category;
- an experience level category;
- a target muscle category; and
- a workout type category.

7. Method (100) according to any one of the preceding claims, **characterized in that** said grouping criteria for determining said fitness profile groups (52) comprise distance-based clustering algorithms, such as K-means and qt-clustering.

8. Method (100) according to any one of the preceding claims, **characterized in that** said step of generating (150) a multimedia content recommendation (Cⱼ) comprises the sub-step (151) of selecting a multimedia content (C^{*}ⱼ) based alternatively:
on the probability weighted by said number of combinations; and/or
on a random selection of a multimedia content (C^{*}ⱼ).

9. Method (100) according to any one of the preceding claims, **characterized in that** said multimedia content (Cⱼ) comprises at least one of:
- videos, such as musical exercise training videos;
- audio files;
- podcasts;
- videos with a single trainer instructing the workout;
- class videos, with participants; and
- audio or video description of the exercise.

10. Method (100) according to any one of the preceding claims, **characterized**
**in that** said control unit (2) is a cloud server, remotely connected with said exercise devices (3; 31, 32,..., 3n), and
**in that** said operational data (4) is stored in a database or storage unit (5), connected to said control unit (2), in which said fitness profile groups (52) and said plurality of category combinations (51) are stored,
wherein said database or storage unit (5) is located on a remote server.

11. Method (100) according to any one of the preceding claims, **characterized**
**in that** said step of generating a recommendation (150) comprises the transmission of said recommendation through a data exchange interface (22) of said control unit (2),
wherein said data exchange interface (22) is connected to said database or storage unit (5).

12. Method (100) according to any one of the preceding claims, **characterized in that** said communication device (311, 312,..., 31n) comprises a display.

13. Method (100) according to any one of the preceding claims, **characterized in that** one or more exercise devices (3; 31, 32,..., 3n) comprise:
gym machines (31, 32,..., 3n), such as stationary bikes, treadmills, rowing machines and the like; and/or
wearable devices, such as smartwatches, heart rate straps, fitness trackers and the like.

14. System (1) for recommending personalized fitness content, comprising:
one or more exercise devices (3; 31, 32,..., 3n) for performing exercises;
a control unit (2), comprising
a processing module (21),
a data exchange interface (22), for allowing data input, connected to said processing module (21) and for transmitting said data, and
a memory (23) containing instructions that, when executed by said processing module (21), cause said control unit (2) to execute the steps of the method (100) according to any one of the preceding claims;
a database or storage unit (5), operatively connected to said control unit (2), in which said fitness profile groups (52) and said plurality of category combinations (51) are stored;
wherein said control unit (2) is connected to said exercise devices (3; 31, 32,..., 3n), for acquiring said operational data acquired within a time interval (*ΔT*) from one or more users (Uₖ).

15. System (1) according to the preceding claim, **characterized in that** said recommended content (Cⱼ) is displayed via the communication device (311, 312,..., 31n) of the respective exercise device (3; 31, 32,..., 3n).

16. System (1) according to any one of claims 14 or 15, **characterized in that** said data exchange interface (22) is an Application Programming Interface (API) module.

17. System (1) according to any one of claims 14 - 16, **characterized in that** said operational data (4) comprises:
- training data relating to one or more users (Uₖ) and multimedia content usage data (Cⱼ) by said users (Uₖ); and/or
- information about the health of the users (Uₖ); and/or
- preferences and training objectives of each of said users (Uₖ).

18. System (1) according to any one of claims 14 - 17, **characterized in that** said processing module (21) executes said method (100) by means of algorithms,
wherein said algorithms comprise at least one artificial intelligence algorithm.

19. Computer program comprising instructions that, when the program is executed by a computer, cause the computer to perform the steps of the method (100) according to any one of claims 1-13.

20. Computer-readable storage medium comprising instructions that, when executed by a computer, cause the computer to perform the steps of the method (100) according to any one of claims 1-13.
